# EUROPEAN PATENT SPECIFICATION

(11) **EP 0 439 513 B1**
(45) Date of publication and mention of the grant of the patent: **13.03.1996**
(21) Application number: 89912069.5
(22) Date of filing: 17.10.1989
(51) Int. Cl.: A61K 33/38, A61K 31/635, A61K 31/28

(54) **COMPOSITION FOR INHIBITING TRANSMISSION OF AIDS**
Zusammensetzung zur Verhinderung der übertragung von AIDS.
COMPOSITION INHIBITANT LA TRANSMISSION DU SIDA

(30) Priority: 18.10.1988 US 262165
(43) Date of publication of application: 07.08.1991
(60) Divisional application: 94203171.7
(73) Proprietor: The Trustees of Columbia University in the City of New York, New York, NY 10026-6699 (US)
(72) Inventor: MODAK, Shanta, M., River Edge, NJ 07661 (US); FOX, Charles, L., Jr., deceased (US)
(74) Representative: de Bruijn, Leendert C.
(86) International application number: PCT/US89/04642
(87) International publication number: WO 90/04390

(56) References cited:
- EP-A- 0 287 204
- US-A- 3 761 590
- US-A- 4 415 565
- CHEMICAL ABSTRACTS, vol. 102, p. 304, 1985, abstract no. 128281; BOUDOUMA, M. et al.: "A simple method for evaluation of antiseptic and disinfectant virucidal activity"
- CHEMICAL ABSTRACTS, vol. 101, p. 8, 1984, abstract no. 221999p; QUERO, A.M. et al.: "Determination of the virucidal activity of antiseptics by a gel filtration method"
- CHEMICAL ABSTRACTS, vol. 83, p. 38, 1975, abstrct no. 71620b; CHANG et al.: "In vitro activity of silver sulfadiazine against Herpesvirus hominis"

## Description

### Background of the Invention

The present invention relates to composition for inhibiting the transmission of Acquired Immunodeficiency Syndrome (AIDS).

AIDS is a fatal catastrophic disease that presently infects millions of people worldwide. Although initially concentrated in central Africa and in certain high risk groups in other geographic areas including the United States, AIDS is now spreading to other areas and is appearing in individuals who are not members of the recognized risk groups. As a result, major efforts are being made to develop methods of preventing the transmission of AIDS, methods of curing AIDS once contracted, and methods of ameliorating the symptoms of AIDS. To date, however, AIDS has proven difficult to treat or prevent.

AIDS is caused by a virus. This virus has been referred to by a number of names in the literature, including HIV (human immunodeficiency virus), LAV (lymphadenopathy-associated virus), ARV (AIDS-related virus) and HTLV-III (human T-cell leukemia virus-III). For simplicity, the virus causing AIDS will be referred to herein as the AIDS virus.

It is generally known that viruses can be divided into two groups based upon the nature of the virus' genetic material. Some viruses are DNA viruses, that is there genetic material is deoxyribonucleic acid, while others are RNA (ribonucleic acid) viruses. The RNA viruses can further be divided into two groups, those in which replication of the viral genome proceeds by making an RNA copy directly from the RNA genome and those in which a DNA intermediate is involved. This latter type off RNA virus is called a retrovirus.

The AIDS virus is a retrovirus. Thus, like other retroviruses, it has an enzyme called reverse transcriptase (or RNA-dependent DNA polymerase) which catalyzes transcription of viral RNA into double helical DNA. This DNA sequence is integrated into the genome of the infected cell where it is known as a provirus. Subsequent transcription of this provirus by the transcription mechanism of the infected cell produces new viral RNA for packaging into new virus particles.

Because the AIDS virus may lie dormant in an infected cell in the form of a provirus for extended periods of time, it has been difficult to establish the precise routes by which AIDS is spread. It is known, however, that AIDS can be transmitted to a person by transfusing that person with blood containing the AIDS virus. AIDS can also be transmitted to a person through homosexual or heterosexual intercourse with a partner infected with the AIDS virus. Transmission of the AIDS virus is facilitated by preexisting sexually transmitted diseases (STD's) other than AIDS, for example gonorrhea. Further, scientists suspect that the AIDS virus is spread easily during sexual intercourse due to tearing of tissue which would abet entry of the AIDS virus into the blood stream.

In response to the growing threat of AIDS transmission, the use of condoms during sexual intercourse has been suggested as a means of preventing transmission of the AIDS virus. Improper use of condoms, or their perforation during intercourse renders them only partially effective. Accordingly, there is a pressing need for a better method of inhibiting the transmission of the AIDS virus in humans during sexual intercourse and during surgical procedures on infected patients. It is an object of the present invention to provide such a method.

### Summary of the Invention

The present invention provides an inexpensive, easily available and convenient topical composition for inhibiting transmission of sexually transmitted diseases including AIDS and hepatitis characterized in that the composition comprises an effective antiviral amount of a biguanide and a silver salt in an amount with which the biguanide exhibits synergistic antiviral activity, for inhibiting the transmission of the AIDS virus in humans for example, as a result of sexual intercourse. The invention relies upon a dual mode of action of particular compounds and combinations thereof which results in a rapid killing action within minutes. These compounds are effective to reduce the infectivity of the AIDS virus and also kill the causative organisms of many other STD's after short exposure. The method of the invention is therefore useful to reduce the immediate risk of AIDS transmission. It also reduces future risk of AIDS transmission by eliminating STD causing organisms which increase the risk of AIDS.

Silver salts, such as silver sulfadiazine (AgSD), are among the compounds found to be effective antiviral agents against retroviruses including the AIDS virus. Such materials had previously been recognized as antibacterial agents useful in treating burns in man and animal. C.L. Fox, Jr., U.S. Patent No. 3,761,590. AgSD has also been shown to be effective against certain viruses such as herpes simplex and herpes zoster and against the causative organisms of many STD's including Candida albicans, Treponema pallidum and gonorrhea. U.S. Patent No. 4,415,565 of Wysor shows further antiviral activity of AgSD against certain RNA viruses, but none of these are retroviruses. Thus, while AgSD is a well studied material, there was no basis to expect that it would have activity against the AIDS retrovirus which has proven so difficult to inhibit or destroy.

In the European patent application No. 88301620.6 filed February 25, published as EP No. 0287204 (thereby forming a prior right under Article 54.3 EPC) a method of inhibiting the transmission of AIDS virus in humans upon sexual intercourse comprising topically applying an effective antiviral amount of silver sulfadiazine to a sexual canal, such as a vaginal canal or an anal canal, of a human prior to or during sexual intercourse is described. No use of other active ingredients is disclosed. A further embodiment of the method comprises application of the silver sulfadiazine as a component of a coating on a condom. A composition for inhibiting the transmission of AIDS virus in humans upon sexual intercourse which comprises an effective antiviral amount of silver sulfadiazine is also described.

Biguanides, such as chlorhexidine, have also been found to be effective when used at sufficiently high levels as inhibitors of the AIDS virus.

We have found that combinations of these compounds with each other and with other antibacterial agents lead to an unexpected enhancement of the antiviral activity of AgSD and also in a rapid killing action. Specifically, AgSD in combination with chlorhexidine, a broad spectrum antibacterial, is substantially more effective for reducing the infectivity of the AIDS virus than AgSD alone, despite the fact the chlorhexidine alone has no effect on infectivity of AIDS virus under the same conditions. Increased effectiveness was also noted for combinations of AgSD with detergents such as deoxycholate.

In view of these findings, the invention contemplates inhibiting the transmission of AIDS comprising topically applying an effective antiviral amount of biguanide and a silver salt such as silver sulfadiazine. Other agents such as deoxycholate may also be used. The composition is advantageously administered to a sexual canal of a human prior to or during sexual intercourse. This application can be carried out by introducing a cream or foam into the sexual canal, or by coating the inhibitory composition onto a condom or other device that is inserted into the sexual canal.

### Brief Description of the Figure

Fig. 1 is a graph of the rate of incorporation of radiolabeled thymidine by hepatitis B virus following exposure of the virus to AgSD alone or in combination with other agents.

### Detailed Description of the Invention

As noted above, the composition of the present invention is effective to inhibit the transmission of AIDS virus in humans and other mammals which applied topically in an effective antiviral amount. The composition comprises a biguanide in combination with other active ingredients.

As used in this application, the term sexual canal refers to either a vaginal or an anal canal.

The antiviral composition used in the method of the invention comprises biguanide, such as chlorhexidine or a salt thereof.

The composition also includes a silver salt. While the examples hereinbelow use one specific silver salt, AgSD, other silver salts may also be used. Other suitable silver salts include silver acetate, silver benzoate, silver carbonate, silver chloride, silver iodate, silver iodide, silver lactate, silver laurate, silver nitrate, silver oxide, silver palmitate, and silver salts of proteins.

The antiviral composition of the invention preferably also comprises one or more additional ingredients which enhance the antiviral effectiveness of the silver salt. Thus, the antiviral composition may contain detergents such as deoxycholate or benzalkonium chloride. Suitable salts of these materials may also be used.

The antiviral composition may also include other materials which are effective against STD-causing organisms which will reduce the long term risk of AIDS infection. Examples of such materials include nonoxynol, which is effective against gonococcus and quinolones which are effective against numerous STD-causing organisms. It should be noted that chlorhexidine and the detergents noted above are also effective against a variety of STD-causing organisms, including herpes simplex virus (HSV) and Candida albicans.

The antiviral compositions for use in the invention can be applied as (a) a dispersion in a water-dispersible hydrophilic carrier; (b) as a dispersion in a substantially water insoluble carrier; (c) as a dispersion in a semi-soft or cream-like water-dispersible or water-soluble oil-in-water emulsion carrier; or (d) as a dispersion in an aqueous sucrose carrier, e.g. an approximately 25%-50% by weight aqueous sucrose solution, Specific examples of formulating silver sulfadiazine in various carriers are provided in U.S. Patent No. 3,761,590 which is incorporated herein by reference. The carrier will preferably contain from about 0.1 to about 10% by weight of the silver salt and up to 2% of other active agents.

The antiviral composition useful in the method of the invention can be contained in a squeezable tube having an applicator nozzle. This facilitates topical application of the composition to the sexual canal prior to intercourse by inserting the nozzle into the sexual canal and squeezing the tube to force the antiviral composition into the sexual canal. Alternatively, the antiviral can be applied with any of various known applicators for delivering drugs into a sexual canal. The antiviral composition can also be topically applied during sexual intercourse by coating the penis itself or coating a condom with a lubricant material, such as K-Y Jelly (Johnson & Johnson), that contains the silver salt.

The antiviral composition of the invention may also be introduced into the sexual canal as a coating on a device intended for insertion in the sexual canal. Examples of such devices include condoms, medical gloves, and diaphragms. Such devices may be coated or impregnated with the antiviral composition by spraying the completed device or by incorporating the antiviral composition during manufacture. Specific techniques for preparing the devices are described in U.S. Patent Application Serial No. 154,920, filed February 11, 1988, and its combination-in-part filed October 14, 1988, both of which are incorporated herein by reference.

The experimental results which demonstrate the effectiveness of the claimed method are set forth below. These tests involve the AIDS virus, a recognized model system for the AIDS virus or a recognized STD organism. Further, although the tests with the AIDS virus itself are necessarily in vitro tests in view of the catastrophic consequences of AIDS, these in vitro tests are highly predictive of and correlate with in vivo efficacy. They thus support the surprising finding that compositions containing biguanides with silver salts can be used to inhibit transmission of AIDS as a result of sexual intercourse.

### Comparative Example 1

The effectiveness of AgSD against the AIDS virus J1 in vitro was assessed by testing the infectivity of samples of HTLV-III in H9 cells after exposure to AgSD for 10 minutes. Due to the relatively low titers achievable with the AIDS virus, it was necessary to devise means for separating the bulk of the AgSD from the virus to be assayed. After a number of preliminary experiments, it was found that the best method of those investigated was to rapidly pass the AgSD/AIDS virus mixture over a Sephadex G-25M column, recover the AIDS virus containing void volume and precipitate the virus using polyethylene glycol (PEG).

To determine recovery of the virus using this method, a control preparation containing virus but no AgSD was similarly processed.

It was also necessary to confirm that this procedure was effective to remove all of the AgSD. This was accomplished using "Stop Controls". This involved processing AgSD alone through the column, precipitating the same fraction with PEG and then adding active AIDS virus to the precipitate. If the titer of the stop control had been similar to the control preparation containing virus but no AgSD it would have indicated that little or no AgSD was present in the precipitate. In fact, however, the titer was substantially lower in the stop controls (Samples 4 and 6) than in the corresponding test samples without silver sulfadiazine (Samples 1 and 2). This indicates that some of the silver sulfadiazine is not being separated. While this means that virus killing occurred over a longer period than the ten minute contact time, it also suggests that the virucidal activity is fairly strong to persist even at the reduced levels.

The specific tests conducted are summarized in Table 1. For each sample to which virus was added initially, the virus sample was a stock solution prepared from a 10,000 fold concentrate of HTLV-III obtained from Bionetics Research. This material was diluted 1:10 with Conditioned Infection Medium (CIM) to form a stock solution with an actual virus titer of 10^{5.5}/ml. Two AgSD stock preparations were also prepared, a 1% by weight in 50% by weight aqueous sucrose preparation and an 0.5% by weight in 25% by weight aqueous sucrose preparation.

To conduct the tests, 60 µl aliquots of the virus stock were placed in microfuge tubes as samples 1-3 and 6 as indicated in Table 1. This was mixed with 540 µl of the respective AgSD preparations in tubes 3 and 5 and with 540 µl of CIM in tubes 1 and 2. Tubes 4 and 6 each received 600 µl of the respective AgSD preparations, but no virus. Each tube was then mixed with a vortex mixer and allowed to incubate for 10 minutes at room temperature.

To separate the AgSD from the virus, the contents of each tube containing AgSD then centrifuged in a microfuge for 1 minute, and the supernatants were collected. These supernatants and the entire sample of tube 2 were then introduced onto a Sephadex-25M column. The columns used had a fitted disc at the top of the column and a void volume of approximately 1 ml. These columns are normally stored in sodium azide and had been prepared by washing under sterile conditions with 18 successive 4 ml portions of CIM medium on the day prior to the experiment.

Each of the samples was placed on the column until it passed through the fitted disc. The column was then eluted with 4 ml of CIM medium. The first 3 ml of eluent was discarded and the last ml was collected into a sterile microfuge tube containing 0.35 ml of 30% PEG 6000 in phosphate buffer. These tubes were held at 0°C for at least 30 minutes and then centrifuged for 1 minute in a microfuge. The pellets were collected and resuspended in either 0.5 ml CIM (samples 2, 3 and 5) or in an HTLV-III containing medium made by diluting 0.7 parts of the virus stock with 6.3 parts of CIM.

Each of the six samples thus prepared was assayed in quadruplicate with 10-fold dilutions in CIM for its ability to infect H9 cells, This was done by adding 50 µl of a preparation containing 2.4 X 10⁶/ml H9 cells that had been conditioned in CIM for 1 hour at 37°C to each 100 µl of sample or dilution. This culture was fed 25 µl of CIM on days 4, 7 and 10. On day 4, cytotoxicity was evaluated by visual examination of the cultures.

The results of these observations are shown in Table 1. As can be clearly seen, AgSD substantially reduced the infectivity of AIDS virus tested without any observation of cytotoxicity.

### Example 2

The effect of AgSD, chlorhexidine and sodium deoxycholate, both individually and in combination, on the infectivity of the ARV-2 strain of AIDS virus was tested in H9 cells using lower concentrations of drug such as can be practically coated onto a glove or condom or other device. These concentrations were below the level that produced substantial observable cytotoxicity, even during incubation with the virus, and yet were effective at killing the virus.

A stock solution of virus containing 3 to 5 X 10⁴ infectious virus particles/ml was preincubated with the various drugs as indicated in Table 2 for 15 minutes. The virus sample was then diluted 4-fold in order to reduce the concentrations of the drugs below levels toxic to H9 cells (see Example 3 below) and mixed with 250,000 H9 cells in a total volume of 1 ml. After 24 hours, the cells were assayed to determine the percentage of the culture expressing viral antigen. This time interval was selected as it allows for only a single round of viral infection to have occurred such that the number of cells infected was a direct reflection of the number of infectious virions present in the original sample.

As can be seen from Table 2, AgSD alone at these low concentrations was only slightly effective, but better results were obtained when AgSD was used in combination with either sodium deoxycholate and chlorhexidine. Of particular significance is the marked reduction in infectivity observed for the combination of AgSD (5 µg/ml) and chlorhexidine (5 µg/ml) since chlorhexidine (10 µg/ml) did not itself reduce viral infectivity.

### Example 3

The toxicity of the various agents used in the antiviral compositions of the invention to human T₄-lymphocytes (H9 cells and marophages which are the carriers of the AIDS virus may be relevant to the effectiveness of a drug. This is because killing these cells when present in semen or vaginal fluids may lead to release of virus making it more susceptible to the effects of the drug. With this in mind, the effect of short exposure (10 minutes) of AgSD and other drugs on H9 cells was tested by treating a suspension of H9 cells (1.6 X 10⁶/ml in HBSS) with 50 and 100 ml/ml of each drug or drug combination. After incubating for 10 minutes, the cells were washed twice in thirty volumes of HBSS; resuspended in RPMI 10% FCS - NaPyruvate and plated into 24 well plates at 4 x 10⁵ cells/ml. Cell viability was determined after 24 hours and is reported as numbers of viable cells per ml and viable percentage (live cells/live cells + dead cells) in Table 3A. As can be seen, each of the agents tested kills some of the cells, although the most significant killing is observed for 100 µl/ml AgSD and the combination of AgSD and sodium deoxycholate.

The effectiveness of killing of macrophages was also tested as shown in Table 3B. In the experiment, peritonial normal mouse macrophages were enriched by attaching to petri dishes and adjusted to a cell concentration of 5 to 10 x 10⁶/ml. 0.1ml aliquots of this suspension were plated in microtiter plates and 10µ and 5µ of each of four samples was added. The control plate received PBS only. After 20 minutes of incubation in a CO incubator, the cells were tested for viability using tryphan-blue dye. The percent kill is shown in Table 3B.

### Example 4

In vivo tests were performed using Rauscher Leukemia Virus (RLV), a recognized retrovirus model (see, e.g., Nature 323, 467-469 (1986); Rupecht et al., Proc. Nat'l. Acad. Sci. USA 82, 7733-7737 (1985)) which is used by the FDA in testing drugs for use in treating AIDS. RLV was introduced into Balb/CICR mice in which it infects the spleen. The level of virus infectivity was quantified by determining the weight increase of the mouse spleen after 20 days from infection.

A preliminary experiment was first carried out to determine the effect of the drugs to be tested on the spleen. Nine sets of five mice each (6 week old female mice) received 0.25 ml injections into the tail vein of one of an extract of a glove treated with one of the following solutions:
1. Silver Sulfadiazine (2%)
2. Sodium Deoxycholate (2%)
3. Chlorhexidine (2%)
4. Silver Sulfadiazine (1%) + Sodium Deoxycholate (1%)
5. Silver Sulfadiazine (1%) + Chlorhexidine (1%)
6. Fusidic Acid (2%)
7. Fusidic Acid (1%) + Chlorhexidine (1%)
8. Saline incubated glove
9. Saline-no glove
Each treatment was prepared by incubating 1.5 ml Dulbecco's Phosphate Buffered Saline (PBS) for 10 minutes at 37°C in the finger tip of a latex glove. After incubation, as much as possible of the material was removed from the glove. 0.4 ml of PBS was then introduced into the glove and this was the sample which was introduced into the animals. The animals that did not receive a clean stick during the injection were excluded from the study. Thus two of the groups only had four animals each that were considered.

Eight days after injection each of the animals was sacrificed and the spleen weights determined for each animal. No increase in spleen weight was observed in any of the groups.

An additional eleven groups of 5 mice each were then used to test the effectiveness of these same compounds against infectivity of RLV. Each treatment was prepared by incubating 0.4 ml sterile PBS containing RVB3 (a strain of RLV) for 10 minutes in a glove tip which had previously had one of drugs or straight PBS incubated in it as described above. Three additional controls, a PBS containing glove with no virus, a virus sample not incubated in a glove, and a PBS sample not incubated in a glove were also run. The mice in this case were sacrificed 20 days after injection and spleen weights determined as shown in Table 4. Each of the materials tested showed a substantial reduction in virus infectivity.

### Example 5 .

The combination of AgSD with chlorhexidine and deoxycholate was also found to be particularly effective against several STD-causing organisms. As shown in Tables 5A and 5B silver sulfadiazine in combination with chlorhexidine or sodium deoxycholate is particularly effective against Candida albicans. Similarly, these combinations are effective to kill Gonococcus (Table 6) and herpes virus (Tables 7A and 7B).

### Example 6

The effect of AgSD in combination with chlorhexidine or sodium deoxycholate on DNA synthesis by Hepatitis B virus was studied by measuring the rate of incorporation of radiolabeled thymidine. As a result, it was found that the AgSD in combination with either chlorhexidine or sodium deoxycholate (Fig. 1) interferes with the RNA-dependent DNA polymerase of Hepatitis B virus.

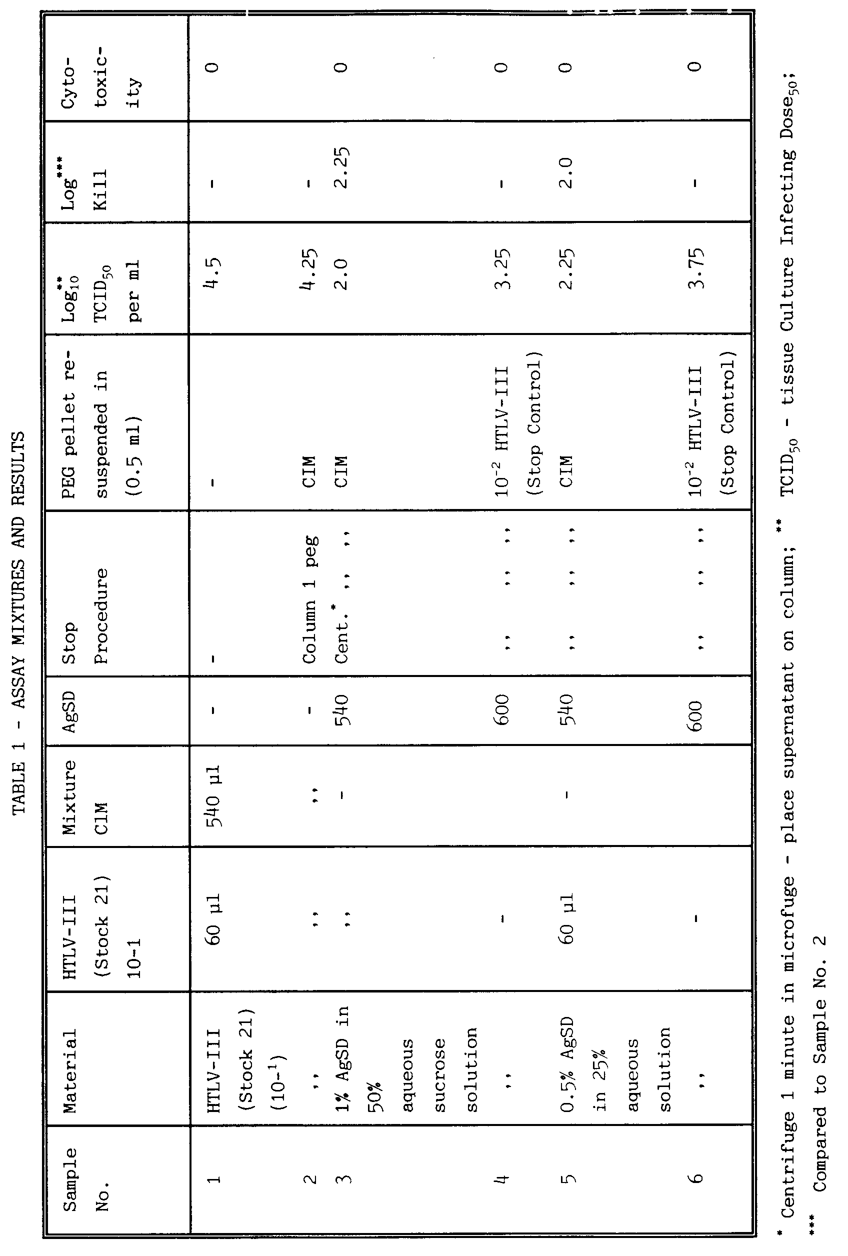

**TABLE 2**

| Drug During | | Final (Drug) µg/ml | % Infection | % Infection v. Control |
|---|---|---|---|---|
| Incubation | (µg/ml) | | | |
| chlorhexidine (CHA) | 10 | 2.5 | 3.35 | 108 |
| Sodium deoxycholate (NaDC) | 40 | 10.0 | 3.35 | 108 |
| AgSD | 10 | 2.5 | 2.95 | 95 |
| AgSD + | 10 | 2.5 + | 2.85 | 92 |
| NaDC | 40 | 10.0 | | |
| AgSD + | 5 + | 1.25 + | 2.45 | 72 |
| CHA | 5 | 1.25 | | |

**TABLE 3a**

| | Viable cells/ml | | % Viable |
|---|---|---|---|
| * AgSD | 50 4 x 10⁵ | cells in terrible condition | 37 |
| | 100 5 x 10⁴ | cells in terrible condition | 0 |
| CHA | 50 1.5 x 10⁶ | cells in terrible condition | ⁻3 |
| | 100 2.5 x 10⁵ | | 20 |
| NaDC | 50 1.2 x 10⁶ | | 73 |
| | 100 2.0 x 10⁶ | | 44 |
| AgSD | 50 1.5 x 10⁴ | | 0 |
| + CHA | 50 | | |
| H₂O | 3.1 x 10⁶ | | 89 |
| Cells alone | 3.0 x 10⁶ | | 88 |

| | | | |
|---|---|---|---|
| * AgSD → insoluble. In an attempt to remove drug cells were spun at 200 g for 15 sec (including acceleration and deceleration time) → Cells pipetted off, then washed two times | | | |
| ** live cells live & dead | | | |

**TABLE 3B**

| Results Rate of Killing of Macrophage by Drugs | |
|---|---|
| | % Kill |
| Control | 36 |
| AgSD (100 µg) | 100 |
| CHA (100 µg) | 100 |
| AgSD + CHA (50 µg + 50 µg) | 85 |

**TABLE 4**

| | Results | | |
|---|---|---|---|
| Drug in glove | Concentration of Drug in coating solution (%) | Weight of Spleen (mg) (average of 6 animals) | Weight increase from control (mg) |
| Silversulfadiazine | 2 | 106 | 20 |
| Deoxycholate | 2 | 109 | 23 |
| Chlorhexidine | 2 | 234 | 148 |
| Silver sulfadiazine + deoxychlolate | 1+1 | 115 | 29 |
| Silver sulfadiazine + chlorhexidine | 1+1 | 103 | 17 |
| Fusidic acid | 2 | 107 | 21 |
| Fusidic acid + chlorhexidine | 1+1 | 319 | 23 |
| Control glove + PBS medium | | 86 | 0 |
| No glove - only PBS medium | | 86 | 0 |
| Control glove + RVB3 | | 1,627 | 1,541 |
| No glove + RVB3 | | 1,280 | 1,194 |

**TABLE 5A**

| Rate of killing of Candida-albicans by silver sulfadiazine and other agents on short exposure | | |
|---|---|---|
| Drug | Concentration | Colony Counts in culture (10 min. incubation) |
| Silver sulfadiazine | 100 | 10,000 |
| Chlorhexidine | 100 | 30 |
| Deoxycholate | 1,000 | 8,000 |
| AgSD + Chlorhexidine | 50+50 | 0 |
| AgSD + Deoxycholate | 100+100 | 20 |
| Nonoxynol | 0,2% | >50,000 |
| Control | | >50,000 |
| 3 ml of Saboraud broth containing 10⁵ organisms of Candida albicans were incubated with the above drugs. Aliquots were removed at 5 and 10 minutes and were subcultured. | | |

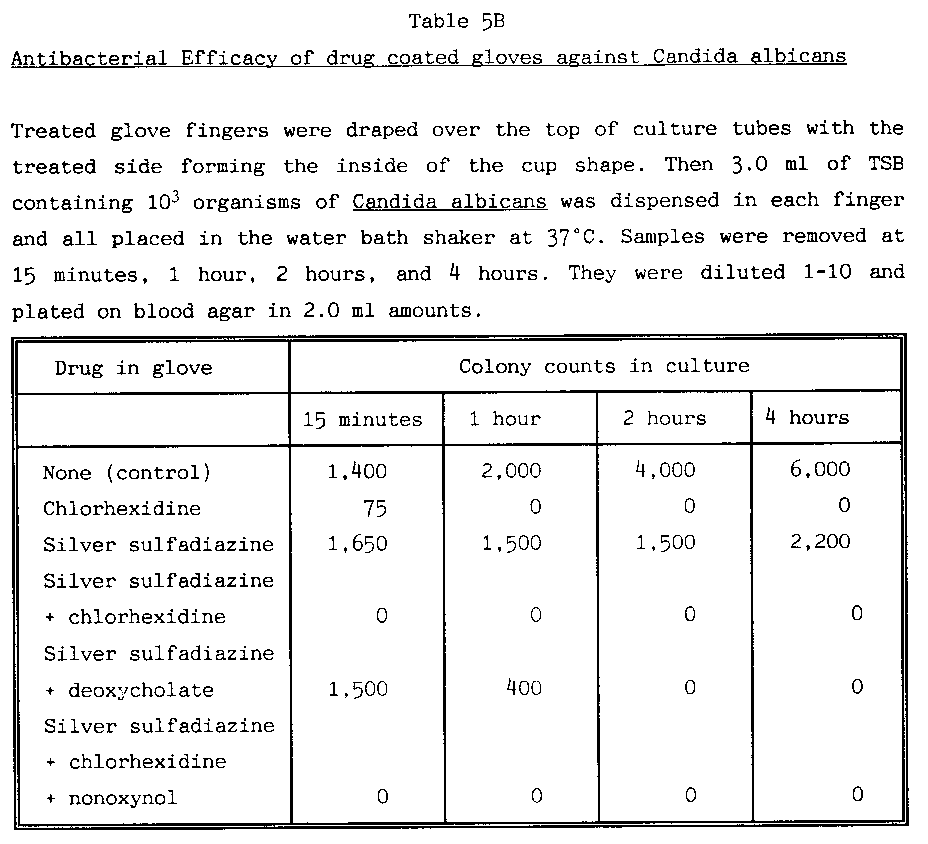

**TABLE 6**

| Killing of Gonococcus by silver sulfadiazine and other agents | | | |
|---|---|---|---|
| Drugs | µg/ml | Colony counts in culture | |
| | | 5 minutes | 10 minutes |
| AgSD | 100 | 4,000 | 2,000 |
| Deoxycholate | 1,000 | 12,000 | 4,000 |
| Chlorhexidine | 100 | 2,000 | 10 |
| Nonoxynol | 0,1% | 40 | 70 |
| AgSD+chlorhexidine | 50+50 | 0 | 0 |
| AgSD+deoxycholate | 100+1,000 | 10 | 0 |
| None (control) | | > 50,000 | > 50,000 |
| Drugs were suspended in 5 ml of cultures containing 105 organisms of Gonococcus and incubated. Aliquots were removed at 5 and 10 minutes intervals and subcultured for colony counts. | | | |

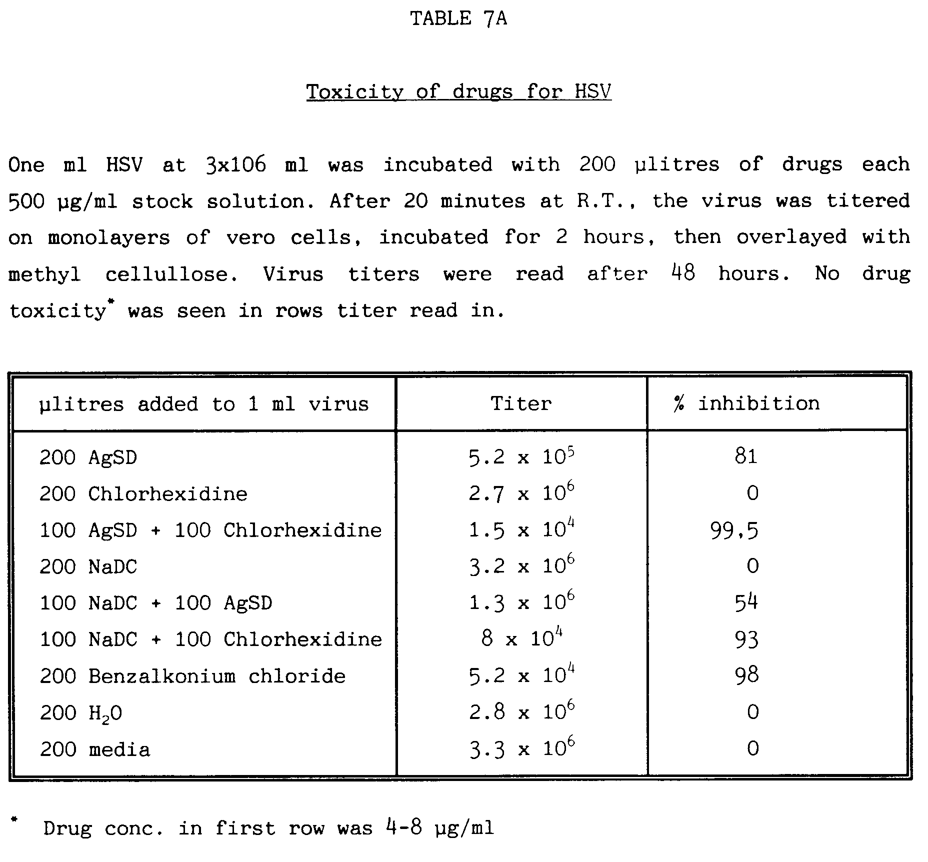

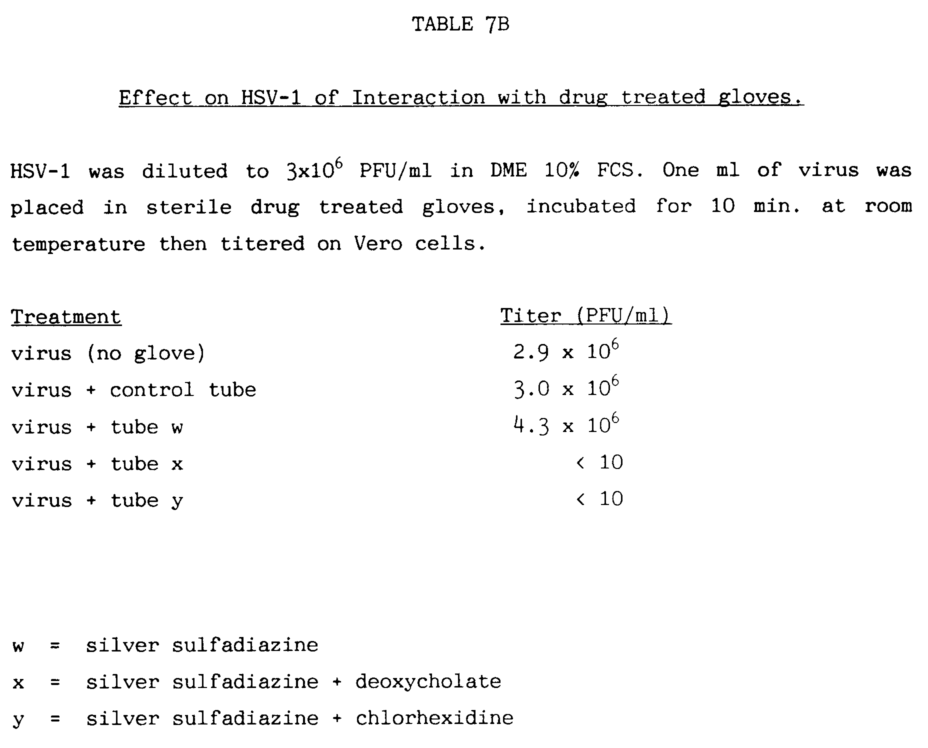

## Claims

1. A topical composition for inhibiting transmission of sexually transmitted diseases including AIDS and hepatitis characterized in that the composition comprises an effective antiviral amount of a biguanide and a silver salt in an amount with which the biguanide exhibits synergistic antiviral activity.

2. A topical composition according to claim 1, **characterized** in that the biguanide is chlorhexidine or a salt thereof.

3. A topical composition according to claims 1-2, **characterized** in that the silver salt is selected from among silver sulfadiazine, silver acetate, silver benzoate, silver carbonate, silver chloride, silver iodate, silver iodide, silver lactate, silver laurate, silver nitrate, silver oxide, silver palmitate, and silver salts of proteins.

4. A topical composition according to claim 3, **characterized** in that the silver salt is silver sulfadiazine.

5. A topical composition according to claims 1-4, **characterized** in that the composition further comprises a detergent.

6. A topical composition according to claim 5, **characterized** in that the detergent is sodium deoxycholate.

7. A topical composition according to claims 1-6, **characterized** in that the composition is a dispersion in a water dispersible hydrophilic carrier.

8. A topical composition according to claims 1-7, **characterized** in that the composition is a dispersion in a water dispersible hydrophilic carrier.

9. A topical composition according to claims 1-7, **characterized** in that the composition is a dispersion in a semi-soft or cream-like, water dispersible or water soluble oil-in-water emulsion.

10. A topical composition according to claims 1-7, **characterized** in that the composition is a dispersion in an aqueous sucrose solution.

11. A topical composition according to claims 3-10, **characterized** in that the composition comprises 0.1 to 10 percent by weight of the silver salt.

12. A device for insertion in a sexual canal, **characterized** in that the device is coated with a composition according to any of claims 1-11.

13. A device for insertion in a sexual canal, **characterized** in that the device is impregnated with a composition according to any one of claims 1-11.

14. A device according to claims 12-13, **characterized** in that the device is a condom.

## Patentansprüche

1. Topische Zubereitung zur Verhinderung der Übertragung von sexuell übertragenen Krankheiten einschließlich AIDS und Hepatitis, dadurch gekennzeichnet, daß die Zubereitung eine antiviral wirksame Menge eines Biguanids und ein Silbersalz in einer Menge enthält, mit der das Biguanid eine synergistische antivirale Wirkung zeigt.

2. Topische Zubereitung gemäß Anspruch 1, dadurch gekennzeichnet, daß das Biguanid Chlorhexidin oder ein Salz desselben ist.

3. Topische Zubereitung gemäß einem der Ansprüche 1 oder 2, dadurch gekennzeichnet, daß das Silbersalz aus Silbersulfadiazin, -acetat, -benzoat, -carbonat, -chlorid, -jodat, -jodid, -lactat, -laurat, -nitrat, -oxid und -palmitat sowie Silbersalzen von Proteinen ausgewählt ist.

4. Topische Zubereitung gemäß Anspruch 3, dadurch gekennzeichnet, daß das Silbersalz Silbersulfadiazin ist.

5. Topische Zubereitung gemäß einem der Ansprüche 1 - 4, dadurch gekennzeichnet, daß sie zusätzlich ein Detergenz enthält.

6. Topische Zubereitung gemäß Anspruch 5, dadurch gekennzeichnet, daß das Detergenz Natriumdesoxycholat ist.

7. Topische Zubereitung gemäß einem der Ansprüche 1 - 6, dadurch gekennzeichnet, daß die Zubereitung eine Dispersion in einem in Wasser dispergierbaren hydrophilen Trägerstoff ist.

8. Topische Zubereitung gemäß einem der Ansprüche 1 - 7, dadurch gekennzeichnet, daß die Zubereitung eine Dispersion in einem in Wasser dispergierbaren hydrophilen Trägerstoff ist.

9. Topische Zubereitung gemäß einem der Ansprüche 1 - 7, dadurch gekennzeichnet, daß die Zubereitung eine Dispersion in einer halbfesten oder cremeartigen, in Wasser dispergierbaren oder wasserlöslichen Öl-in-Wasser-Emulsion ist.

10. Topische Zubereitung gemäß einem der Ansprüche 1 - 7, dadurch gekennzeichnet, daß die Zubereitung eine Dispersion in einer wäßrigen Saccharoselösung ist.

11. Topische Zubereitung gemäß einem der Ansprüche 3 - 10,dadurch gekennzeichnet, daß sie 0,1 bis 10 Gew.- % Silbersalz enthält.

12. Vorrichtung zum Einfuhren in eine Sexualöffnung, dadurch gekennzeichnet, daß sie mit einer Zubereitung gemaß einem der Ansprüche 1 - 11 beschichtet ist.

13. Vorrichtung zum Einfuhren in eine Sexualöffnung, dadurch gekennzeichnet, daß sie mit einer Zubereitung gemäß einem der Ansprüche 1 - 11 imprägniert ist.

14. Vorrichtung gemäß einem der Ansprüche 12 - 13, dadurch gekennzeichnet, daß es sich bei der Vorrichtung um ein Kondom handelt.

## Revendications

1. Composition topique empêchant la transnussion de maladies sexuellement transmissibles, y compris le SIDA et l'hépatite, caractérisée en ce que cette composition contient une certaine quantité, efficace contre les virus, d'un biguanide, et un sel d'argent en une quantité telle que le biguanide exerce avec ce sel une activité antivirale synergique.

2. Composition topique conforme à la revendication 1, caractérisée en ce que le biguanide est la chlorhexidine ou l'un de ses sels.

3. Composition topique conforme à l'une des revendications 1 et 2, caractérisée en ce que le sel d'argent est choisi parmi la sulfadiazine argentique, l'acétate d'argent, le benzoate d'argent, le carbonate d'argent, le chlorure d'argent, l'iodate d'argent, l'iodure d'argent, le lactate d'argent, le laurate d'argent, le nitrate d'argent, l'oxyde d'argent, le palmitate d'argent et les sels argentiques de protéines.

4. Composition topique conforme à la revendication 3, caractérisée en ce que le sel d'argent est la sulfadiazine argentique.

5. Composition topique conforme à l'une des revendications 1 à 4, caractérisée en ce que cette composition contient en outre un détergent.

6. Composition topique conforme à la revendication 5, caractérisée en ce que le détergent est du désoxycholate de sodium.

7. Composition topique conforme à l'une des revendications 1 à 6, caractérisée en ce que cette composition est une dispersion dans un véhicule hydrophile dispersable dans l'eau.

8. Composition topique conforme à l'une des revendications 1 à 7, caractérisée en ce que cette composition est une dispersion dans un véhicule hydrophile dispersable dans l'eau.

9. Composition topique conforme à l'une dès revendications 1 à 7 , caractérisée en ce que cette composition est une dispersion dans une émulsion huile-dans-eau soluble ou dispersable dans l'eau et semi-molle ou semblable à une crème.

10. Composition topique conforme à l'une des revendications 1 à 7, caractérisée en ce que cette composition est une dispersion dans une solution aqueuse de saccharose.

11. Composition topique conforme à l'une des revendications 3 à 10, caractérisée en ce que cette composition contient de 0,1 à 10 % en poids de sel d'argent.

12. Dispositif à insérer dans un conduit sexuel, caractérisé en ce que ce dispositif est revêtu d'une composition conforme à l'une des revendications 1 à 11.

13. Dispositif à insérer dans un conduit sexuel, caractérisé en ce que ce dispositif est imprégné d'une composition conforme à l'une des revendications 1 à 11.

14. Dispositif conforme à l'une des revendications 12 et 13, caractérisé en ce que ce dispositif est un préservatif.
